# EUROPEAN PATENT SPECIFICATION

(11) **EP 0 546 819 B1**
(45) Date of publication and mention of the grant of the patent: **27.03.1996**
(21) Application number: 92311235.3
(22) Date of filing: 09.12.1992
(51) Int. Cl.: C12P 1/06, C12P 17/08

(54) **Process for preparation of streptovaricin by fermentation**
Verfahren zur Herstellung von Streptovaricin durch Fermentation
Procédé de préparation de streptovaricine par fermentation

(30) Priority: 09.12.1991 JP 350208/91
(43) Date of publication of application: 16.06.1993
(73) Proprietor: Shin-Etsu Chemical Co., Ltd., Chiyoda-ku Tokyo 100 (JP)
(72) Inventor: Yamazaki, Motohide, San Diego, California 92122 (US); Inoue, Kaname, Miyamae-ku, Kawasaki-shi, Kanagawa-ken (JP)
(74) Representative: Bassett, Richard Simon

(56) References cited:
- EP-A- 0 482 908
- JP-A- 3 219 887
- JP-A- 3 219 888
- US-A- 3 116 202
- Biology of Microorganisms. pub. Prentice-Hall International, Author: T.D.Brock. pages 748 to 749, 1970 and 1984 editions

## Description

### BACKGROUND OF THE INVENTION

### I. Field of the invention

The present invention relates to a process for the preparation of streptovaricins (macrolide antibiotics) by the fermentation of Actinomyces belonging to the genus Streptomyces.

### II. Description of the Prior Art

Five types of streptovaricins are known, designated as A, B, C, D and E. Initially, they drew attention because of their usefulness as an antituberculosis antibiotic. Recently, it has been found that derivatives obtained by chemically modifying the streptovaricins, and particularly, Streptovaricin C, are useful as anti-retroviral agents, anti-cancer agents or the like (e.g., Japanese laid-open patent application No. 110,000/1979).

A known process for the preparation of the streptovaricins comprises fermenting a submerged culture of Streptomyces spectabilis (Japanese Patent Publication No. 3647/1961).

Furthermore, in order to improve productivity by fermentation, there are a method in which a nonionic adsorbent is added to a culture medium for streptovaricin preparation by fermentation (Japanese Patent Application No. 14285/1990), and a preparation method by fermentation described in Journal of Industrial Microbiology, 5, pp. 283-288 (1990) in which an adsorbent is used.

However, in the conventional preparation methods of the streptovaricins by fermentation, the concentration of an accumulated antibiotic in the culture medium is limited. Even if a nonionic adsorbent such as polystyrene is used as in Japanese Patent Application No. 14285/1990, the satisfactory concentration of the accumulated antibiotic cannot be obtained.

It is known, for example from Brock (1984) "Biology of Microorganisms", Prentice-Hall, that a change in the nutrition of Streptomyces may result in a change in the nature of the antibiotic produced.

### SUMMARY OF THE INVENTION

In view of the above-mentioned situations, the present invention has been attained, and an object of the present invention is to provide a novel method for the preparation of streptovaricins by fermentation which comprises adding a nonionic adsorbent to a culture medium so as to positively adsorb and stably recover these streptovaricins, and then carrying out culturing, this method being characterized in that the streptovaricins are allowed to easily move in the culture medium and reach the adsorbent, adsorbed, and recovered to further improve the fermentation productivity of the streptovaricins.

That is, the present invention intends to solve the problems of the above-mentioned conventional methods, and its gist reside in that a process for the preparation of streptovaricins by fermentation comprises culturing Actinomyces belonging to the genus Streptomyces in the presence of a nonionic adsorbent and a vegetable oil to produce the streptovaricins.

According to the present invention, produced streptovaricins can promptly transfer from the surfaces of bacteria to an adsorbent in a culture medium, whereby the streptovaricins which are unstable in an aqueous solution can reach the adsorbent in a stable state. Furthermore, the addition of an emulsifying agent, an emulsion/suspension stabilizer and an organic solvent permits the enhancement of the effects. In these points, the present invention is excellent.

### DESCRIPTION OF THE PREFERRED EMBODIMENT

Actinomyces (a streptovaricin-producing strain) belonging to the genus Streptomyces usable in the process of the present invention includes, for example, Streptomyces spectabilis (available from ATCC under Deposition No. ATCC27465) and its variants. An example of these variant is deposited under Deposition No. FERM BP-3598 in Fermentation Research Institute, Agency of Industrial Science and Technology in Japan.

Suitable for use as the nonionic adsorbents in the present invention are porous fine particles having a large specific surface area and consisting of various synthetic resins, such as, polymers of one or more chemicals selected from the group consisting of aromatic monomers such as styrene and divinylbenzene and acrylic esters. Specific examples include adsorbents composed of styrene-divinylbenzene type synthetic resins, for example, HP-10, HP-20, HP-30, HP-40, and HP-50 (trade name, produced by MITSUBISHI KASEI CORPORATION), Amberlite XAD-2 and XAD-4 (trade names, produced by Rohm and Haas Co.), adsorbents composed of acrylic ester type resins, for example, Amberlite XAD-7 (trade names, produced by Rohm and Haas Co.), and the like. Particularly preferred among these nonionic adsorbents are those having a particle size of from 50 to 1,000 µm, a specific surface area of 50 to 1,000 m/g, and a pore volume of from 0.2 to 1.5 ml/g. Of the above-mentioned commercial products, examples which meet these requirements include HP-20, HP-21 and XAD-4, but they are not particularly restrictive.

The amount of the nonionic adsorbent to be added to the culture medium is preferably from about 0.1 to 20% by weight, more preferably from 1.0 to 10% by weight. In the system in which the amount of the nonionic adsorbent is too large, bacteria are ground at the time of stirring, so that the good growth of the bacterial cells is impossible.

In the present invention, the kinds and amount of the vegetable oil should be remained within the extent which do not have any influence on the culture of the bacteria and the production of the streptovaricins. Specific examples of the vegetable oil include soybean oil, corn oil, rapeseed oil, sesame oil, olive oil, palm oil, teaseed oil, oleic safflower oil, oleic sunflower oil, linseed oil, tung oil, and other vegetable oils containing fatty acids esters. Examples of the fatty acids which can be contained in these vegetable oils include linolenic acid, linoleic acid, and eicosapentanoic acid.

Among these oils, particularly preferred are soybean oil, corn oil, rapeseed oil, sesame oil, and olive oil. The amount of the vegetable oil is from 0.1 to 10% by weight, preferably from 0.2 to 7% by weight based on the total weight of the culture medium. When the amount of the vegetable oil is more than 10% by weight, this vegetable oil easily separates from the culture medium to cover the culture medium, so that the oxygen demand of the bacteria is not satisfied and good results cannot be obtained. When no vegetable oil is added, the productivity of the streptovaricins is lower than when it is added. In this connection, the vegetable oil can be added in the form of soybean meal, peanut meal (which is not defatted), or the like.

In the case that the above-mentioned vegetable oil is used, there can be used an emulsifying agent which can uniformly emulsify the vegetable oil and a thermally stable water-soluble polymer (an emulsion/suspension stabilizer; a thickener) which can prevent the vegetable oil from separating from the culture medium. Examples of the emulsifying agent include glycerol fatty acid ester, sorbitan fatty acid ester, propylene glycol fatty acid ester, sucrose fatty acid ester, and lecithin. Examples of the thermally stable water-soluble polymer include water-soluble polymeric polysaccharides such as welan gum and rhamsan gum. In the case the water-soluble polymer is added, preferred is a water soluble polymer in which a suspension stabilizing effect scarcely deteriorate at the time of heating sterilization of the culture medium.

In the present invention, an organic solvent can be additionally used together with the above-mentioned vegetable oil, emulsifying agent and water-soluble polymer to easily transfer the streptovaricins from the bacteria to the adsorbent and to thereby achieve the improvement of its productivity. Example of the organic solvent include lower alcohols such as methanol, ethanol, propanol, octanol and isopropanol, higher alcohols, higher cyclic alcohols, and a lower ketone such as acetone. The amount of the organic solvent to be added is from 0.1 to 2.0% by weight, preferably from 0.2 to 1.0% by weight based on the weight of the culture medium.

No particular restriction is put on the culture medium and other conditions which are used in the method of the present invention, and the conditions conventionally adopted for the preparation of antibiotics by culturing microorganisms can be used. That is, submerged culture is carried out in an aqueous medium containing a nitrogen source, an assimilable carbon source, and an inorganic salt under aerobic conditions.

As the nitrogen source, any of the inorganic and organic nitrogen sources can be used. Examples of the nitrogen source include organic nitrogen sources such as beef extract, vegetable proteins (e.g., peptone), casein, malt extract, fish meal, cotton meal, Kay Soy (defatted soybean meal), yeast for brewing, corn gluten meal, and corn steep liquor; and inorganic nitrogen sources such as ammonium sulfate, ammonium nitrate, and potassium nitrate.

Examples of the assimilable carbon sources include glucose, dextrin, molasses, starch, maltose, galactose, mannitol, sucrose, and lactose.

Examples of the nutritious inorganic salts include salts which form ions such as sodium, calcium, phosphate, and sulfate. Specific examples thereof include calcium carbonate, potassium phosphate, magnesium sulfate, potassium chloride, sodium chloride, zinc sulfate, ferrous sulfate, manganese sulfate, cobalt chloride, and ammonium molybdate.

During culturing, the pH of the medium is in the range of from about 5.5 to about 7.5, and the temperature is in the range of from about 23°C to 37°C, preferably from 25°C to 30°C. The maximum yield can be obtained by culturing for a period of from about 14 to 17 days.

With the embodiment of the present invention wherein the streptovaricins obtained by the fermentation are adsorbed on the nonionic adsorbent, it is necessary to separate the streptovaricins from the nonionic adsorbent after separation of the nonionic adsorbent from the medium.

The separation of the nonionic adsorbent from the culture medium can be achieved by, for example, filtering the culture medium through a net or the like, or utilizing the difference in specific gravity between the nonionic adsorbent and the culture medium (in the concrete, for example, by putting the culture medium after the fermentation in a sodium chloride solution having a constant concentration, using a centrifugal separator, or the like).

The streptovaricins may be separated from the nonionic adsorbent by washing the separated adsorbent with a suitable organic solvent or a mixed solvent of an organic solvent and water to elute the adsorbed streptovaricins. Examples of the suitable organic solvent include methanol, ethanol, acetone, acetonitrile, ethyl acetate, dichloroethane, chloroform, and mixtures thereof; and mixed solvents of one or more of these organic solvents with water. In order to effectively elute the streptovaricins, it is preferred that the adsorbent on which the streptovaricins are adsorbed be washed first with an aqueous solution having a low organic solvent concentration, and then with an aqueous solution having a high organic solvent concentration.

Among the streptovaricins obtained by the fermentation, Streptovaricin C is the most useful, and therefore it is desirable that during the separation from the adsorbent, Streptovaricin C can be selectively separated. Preferred for this purpose is an acetonitrile-water mixed solvent.

The thus obtained streptovaricins can be further purified, for example, by repeated recrystallization, silica gel column chromatography, and the like.

### Examples

Next, the method of the present invention will be described in more detail in reference to examples. In the following description, "%" means "% by weight".

### Example 1

### A. Culture and maintenance

In this example, a variant of Streptomyces spectabilis ATCC27465 (FERM BP-3598) was cultured and maintained, and the variant was used to prepare Streptovaricin C.

This variant well grew in a standard agar culture medium at a temperature of 27°C. This kind of microorganisms could produce an anthocyanin dye. On an agar culture medium (a standard glucose agar culture medium having the undermentioned composition) prepared by adding 0.65% of glucose to the standard culture medium, colonies were small (1 to 2 mm), convex, and white on the 2nd or the 3rd day. The cultivation was continued, and the colonies changed from a lemon color (on the 3rd to the 5th days) to a red color (on the 6th to the 8th days) (4 to 5 mm) and the surface state of these colonies looked like smooth and glossy chrysanthemum petals. No sporulation was observed.
Standard glucose agar culture medium:

| | |
|---|---|
| Glucose | 0.65% |
| Peptone | 1.00% |
| Beef extract | 0.30% |
| NaCl | 0.50% |
| Yeast extract | 2.00% |
| Agar | 1.50% |

### B. Seed Culture

100 ml of a seed medium having the undermentioned composition were prepared in a 500-ml conical flask with a baffle, and the culture medium was then inoculated with cultured microorganisms of the above-mentioned variant and they were cultured at 27°C at 130 rpm for 72 hours to obtain a flask seed (a seed culture). In this culture medium, the microorganisms grew in the form of fine pellets. After a passage of 72 hours, pellets colored yellow owing to the production of Streptovaricin C and the pH of the culture medium was in excess of 8.0, and saccharide was completely consumed.
Seed Medium:

| | |
|---|---|
| Glucose | 0.625% |
| N-Z amine A | 1.250% |
| (hydrolyzed casein) | |
| Soytone | 0.625% |
| K₂HPO₄ | 0.156% |
| KH₂PO₄ | 0.156% |

### C. Preproduction Culture

Next, 3 liters of a preproduction medium having the undermentioned composition were prepared in a 5-liter fermenter, and this culture medium was then inoculated with 50 ml of the above-mentioned seed culture and microorganisms were cultured at 27°C for 72 hours at an air feed rate of 1.7 vvm at 300 rpm to obtain a preproduction culture. In the preproduction culture in which the good growth of the microorganisms was confirmed, a red color of the bacteria was not observed.
Preproduction Medium:

| | |
|---|---|
| Cornstarch | 2.00% |
| Defatted soybean meal | 1.00% |
| Corn steep liquor | 1.00% |
| Beer yeast | 0.25% |
| KCl | 0.30% |
| CaCO₃ | 0.40% |
| Anti-foaming agent (KM72) | 0.10% |

### D. Main Culture

In a 30-liter fermenter, 18 liters of a main medium was prepared by adding 5.0% of soybean oil to the undermentioned composition, and the thus obtained main culture medium was then inoculated with 900 ml of the above-mentioned preproduction culture, followed by fermentation at 27°C at 150 rpm for a period of from 14 to 17 days. The production of Streptovaricin C was in excess of 2,000 µg/ml, and the satisfactory results were obtained.
Main medium (pH 7.0±0.2):

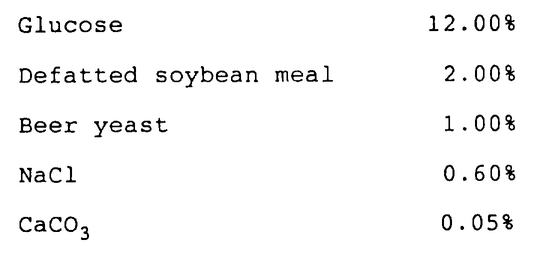

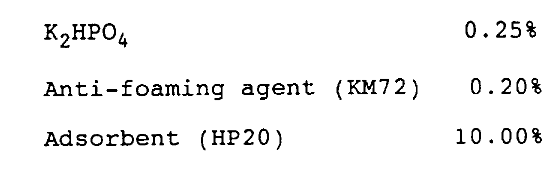

### E. Recovery

After adjusted to a pH of 5 to 6, the culture broth was promptly filtered at a low temperature through a large Buchner funnel to recover the adsorbent from the viscous culture broth.

### F. Extraction

Streptovaricin C was extracted several times from 1,700 g of the thus obtained mixture of the adsorbent and the remaining bacteria by the use of whole 20 liters of methylene chloride.

### G. Concentration and crystallization

The thus extracted solvent was concentrated at 30°C under reduced pressure to 0.2 liter, and at this point of time, ten-fold volume of hexane was added thereto to obtain orange crystals. After collected by filtration, the crystals were washed with hexane again, and then dried under reduced pressure a whole day and night to obtain 68 g of crude Streptovaricin C powder. The purity of this crude Streptovaricin C was so high as to be more than 50%.

### Comparative Example 1

Culturing was carried out by the same procedure as in Example 1 in a system containing no soybean oil in the main culture (the above-mentioned main culture medium composition). As a result, the production of Streptovaricin C was as small as 703 µg/ml, though the employed conditions were all the same with the one exception.

### Examples 2 to 13

The same procedure as in Example 1 was used until a preproduction culture step except that soybean oil was not used, to carry out Examples 2 to 12. Culturing was carried out at 27°C for 16 days in a 500-ml conical flask by the use of a rotary shaker.

In Examples 3 to 6, amounts of soybean oil in Example 1 were changed, and in Examples 7 to 10, vegetable oils other than the soybean oil were used.

In Example 11, lecithin as an emulsifying agent and welan gum as a water-soluble polymer were used in addition to the vegetable oil (soybean oil).

In Example 12, a vegetable oil was replaced with soybean meal (containing 18% of soybean oil; corresponding to a case where 0.9% of soybean oil was used), and ethanol was added as an organic solvent, and lecithin and welan gum were added.

In Example 13, the same procedure as in Examples 2 to 12 was used except that defatted soybean meal in the above-mentioned medium composition for the main culture was replaced with soybean meal (containing 18% of soybean oil; corresponding to a case where 0.9% of soybean oil was used), to carry out the main culture.

The results of Examples 3 to 13 are shown in Table 1 together with the results of Example 1 and Comparative Example 1.

**Table 1**

| Relation between Added Vegetable Oil and Production of Streptovaricin C by Fermentation | | | |
|---|---|---|---|
| Example | Vegetable Oil | Amount | Production of SVC by Fermentation (500-ml conical flask with baffle) |
| Example 1 | Soybean Oil | 5.0% | 2025 µg/ml |
| Comp. Ex. 1 | Soybean Oil | 0.0% | 703 µg/ml |
| Example 3 | Soybean Oil | 0.5% | 1202 µg/ml |
| Example 4 | Soybean Oil | 1.0% | 1517 µg/ml |
| Example 5 | Soybean Oil | 5.0% | 2094 µg/ml |
| Example 6 | Soybean Oil | 10.0% | 1789 µg/ml |
| Example 7 | Corn Oil | 1.0% | 1452 µg/ml |
| Example 8 | Rapeseed Oil | 1.0% | 1354 µg/ml |
| Example 9 | Sesame Oil | 1.0% | 1124 µg/ml |
| Example 10 | Olive Oil | 1.0% | 1231 µg/ml |
| Example 11 | Soybean Oil | 5.0% | 2209 µg/ml |
| | Lecithin | 0.3% | |
| | Welan Gum | 0.3% | |
| Example 12 | Soybean Meal | 5.0% | 1534 µg/ml |
| | Ethanol | 0.3% | |
| | Lecithin | 0.3% | |
| | Welan Gum | 0.3% | |
| Example 13 | Soybean Meal | 2.0% | 1011 µg/ml |

## Claims

1. A process for the preparation of streptovaricins by fermentation which comprises culturing Actinomyces belonging to the genus Streptomyces in the presence of a nonionic adsorbent and a vegetable oil to produce the streptovaricins, wherein, if the oil is solely soybean oil, it is present at at least 0.5%.

2. A process for the preparation of streptovaricins by fermentation which comprises culturing Actinomyces belonging to the genus Streptomyces in the presence of a nonionic adsorbent and a vegetable oil to produce the streptovaricins, wherein the Streptomyces is cultured additionally in the presence of one or more of: an emulsifying agent; a thermally-stable water-soluble polymer; and an organic solvent.

3. A process according to Claim 2 wherein the Streptomyces is cultured additionally in the presence of an emulsifying agent.

4. A process according to Claim 2 or 3 wherein the Streptomyces is cultured additionally in the presence of a thermally stable water-soluble polymer.

5. A process according to any one of claims 2 to 4 wherein the Streptomyces is cultured additionally in the presence of an organic solvent.

## Patentansprüche

1. Verfahren zur Herstellung von Streptovaricinen durch Fermentation, umfassend die Züchtung von zur Gattung Streptomyces gehörigen Strahlenpilzen in Gegenwart eines nicht-ionischen Adsorptionsmittels und eines Pflanzenöls zur Herstellung der Streptovaricine, wobei das Öl, wenn es sich ausschließlich um Sojabohnenöl handelt, in einer Menge von mindestens 0,5% vorhanden ist.

2. Verfahren zur Herstellung von Streptovaricinen durch Fermentation, umfassend die Züchtung von zur Gattung Streptomyces gehörigen Strahlenpilzen in Gegenwart eines nicht-ionischen Adsorptionsmittels und eines Pflanzenöls zur Herstellung der Streptovaricine, wobei der Streptomyces zusätzlich in Gegenwart einer oder mehrerer der folgenden Komponenten: Emulgator, wärmestabiles, wasserlösliches Polymer und organisches Lösungsmittel gezüchtet wird.

3. Verfahren nach Anspruch 2, wobei der Streptomyces zusätzlich in Gegenwart eines Emulgators gezüchtet wird.

4. Verfahren nach Anspruch 2 oder 3, wobei der Streptomyces zusätzlich in Gegenwart eines wärmestabilen, wasserlöslichen Polymers gezüchtet wird.

5. Verfahren nach einem der Ansprüche 2 bis 4, wobei der Streptomyces zusätzlich in Gegenwart eines organischen Lösungsmittels gezüchtet wird.

## Revendications

1. Procédé pour la préparation de streptovaricines par fermentation, qui comprend la culture *d'Actinomyces* appartenant au genre *Streptomyces* en présence d'un adsorbant non ionique et d'une huile végétale pour produire les streptovaricines, dans lequel, si l'huile est seulement de l'huile de soja, elle est présente à au moins 0,5 %.

2. Procédé pour la préparation de streptovaricines par fermentation, qui comprend la culture d'*Actinomyces* appartenant au genre *Streptomyces* en présence d'un adsorbant non ionique et d'une huile végétale pour produire les streptovaricines, dans lequel le *Streptomyces* est cultivé en plus en présence d'un ou plusieurs ingrédients parmi : un agent émulsifiant ; un polymère hydrosoluble thermostable et un solvant organique.

3. Procédé selon la revendication 2, dans lequel le *Streptomyces* est cultivé en plus en présence d'un agent émulsifiant.

4. Procédé selon la revendication 2 ou 3, dans lequel le *Streptomyces* est cultivé en plus en présence d'un polymère hydrosoluble thermostable.

5. Procédé selon l'une quelconque des revendications 2 à 4, dans lequel le *Streptomyces* est cultivé en plus en présence d'un solvant organique.
